# FASCICULE DE BREVET EUROPEEN

(11) **EP 2 862 554 B1**
(45) Date de publication et mention de la délivrance du brevet: **21.06.2017**
(21) Numéro de dépôt: 14189079.8
(22) Date de dépôt: 15.10.2014
(51) Int. Cl.: A61H 15/00

(54) **Appareil de massage avec au moins une tête de massage a rotation excentree**
Massagegerät mit mindestens einem Massagekopf mit exzentrischer Drehung
Massage apparatus having at least one massage head with eccentric rotation

(30) Priorité: 17.10.2013 FR 1360118
(43) Date de publication de la demande: 22.04.2015
(73) Titulaire: SEB S.A., 69130 Ecully (FR)
(72) Inventeur: Giraud, Camille, 69001 Lyon (FR); Fereyre, Régis, 42410 Chavanay (FR); Laranjeira, Laurence, 38440 Moidieu Detourbe (FR); Mandica, Franck, 69340 Francheville (FR)
(74) Mandataire: Guéry-Jacques, Géraldine

(56) Documents cités:
- WO-A1-97/22326
- FR-A1- 2 900 329
- US-A1- 2003 125 648
- US-A1- 2008 183 252
- US-A1- 2010 286 577

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez des personnes désireuses de soigner leur esthétique en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Une demande de brevet US 20100160841 a proposé un corps en forme de poignée revolver sur lequel peut être adapté une tête de massage comprenant cinq têtes de travail liées rigidement à un plateau qui tourne selon un axe de rotation excentré.

Un autre document FR2900329 décrit un appareil de massage motorisé comprenant des éléments de massage rotatifs sous forme de billes et un orgnae d'entrainement des éléments de massage. L'entrainement se fait par friction de chaque élément de massage sur une surface de contact de l'organe d'entrainement.

De tels appareils de massage permettent de réaliser un massage de régions du corps étendues, en revanche ils ne sont pas adaptés pour le massage de régions délicates, de faible étendue et présentant éventuellement un faible rayon de courbure comme cela est le cas pour le visage. Il est donc apparu le besoin d'un appareil de massage plus adapté pour un massage du visage que ceux de l'art antérieur.

Afin d'atteindre cet objectif, l'invention concerne un appareil de massage pour le visage comprenant :
- une tête de massage qui comprend :
   - un élément d'appui qui est destiné à venir contre le visage et qui forme une couronne d'appui définissant, d'une part, une surface d'appui s'inscrivant dans un plan d'appui et, d'autre part, une zone de travail située à l'intérieur de la couronne d'appui,
   - à l'intérieur de la couronne d'appui et dans la zone de travail, au moins une tête de travail qui possède une surface de travail s'étendant en saillie du plan d'appui,
   - des moyens de manoeuvre adaptés pour déplacer en rotation chaque tête de travail selon au moins un axe de rotation décalé par rapport au centre de la surface de travail correspondante,
- et un boitier d'entraînement qui porte la tête de massage et qui comprend un moteur électrique actionnant des moyens d'entrainement adaptés pour transmettre le mouvement du moteur électrique aux moyens de manoeuvre, appareil pour lequel en outre chaque surface de travail s'étend, de manière permanente, en saillie du plan d'appui d'une hauteur H, mesurée entre le sommet de la surface de travail et le plan d'appui.

La conception de la tête de massage de l'appareil selon l'invention est bien adaptée au massage de régions de petite taille telles que celle du visage. En effet la surface d'appui assure la tenue de la peau en délimitant la région du massage de sorte que l'action de chaque tête de travail se trouve concentrée sur la région de la peau située à l'intérieur de la surface d'appui.

Selon une caractéristique de l'invention, la surface d'appui est constituée d'une première matière et chaque surface de travail est constituée d'une seconde matière de rigidité différente de celle de la première matière, la première matière et la seconde matière étant choisies parmi :
- une matière rigide, non déformable sous un effort manuel,
- une matière élastiquement déformable sous un effort manuel.

La mise en oeuvre de deux matières de rigidités différentes pour la surface d'appui et la ou les surfaces de travail permet de bien découpler l'action de la surface d'appui, de celle de la ou des surfaces de travail.

Selon une variante de cette caractéristique, la surface de travail est constituée d'une matière élastiquement déformable. Ce caractère élastiquement déformable de la surface de travail permet d'assurer un bon confort de contact avec la peau de l'utilisateur et une adaptation à la morphologie de la région massée.

Selon une caractéristique de l'invention, chaque surface de travail possède une forme convexe. Cette caractéristique permet d'assurer un massage efficace sans être désagréable ou douloureux.

Avantageusement, chaque surface de travail possède une forme substantiellement sphérique et s'étend, de manière permanente, en saillie du plan d'appui d'une hauteur, mesurée entre le sommet de la surface de travail et le plan d'appui, égale ou supérieure au rayon de courbure de la surface de travail. Selon l'invention cette hauteur de saillie peut être constante ou variable pendant le massage.

Selon une autre caractéristique de l'invention chaque tête de travail comprend une bille qui forme la surface de travail correspondante. La mise oeuvre d'une bille de massage permet de disposer d'une surface de travail de forme sensiblement sphérique.

Selon une variante de cette caractéristique, chaque bille est mobile en rotation sur elle-même. Cette rotation peut être libre ce qui permet à la surface de travail de rouler sur la peau, ou au contraire contrôlée par les moyens de manoeuvre de manière à induire une stimulation particulière de la peau.

Selon une caractéristique de l'invention, chaque surface de travail est rigide.

Selon une autre caractéristique de l'invention, chaque surface de travail est lisse.

Selon encore une autre caractéristique de l'invention, chaque surface de travail est en métal. Cette caractéristique permet par exemple de créer une sensation de fraîcheur lors du contact avec la peau.

Selon encore une variante de cette caractéristique de l'invention, chaque surface de travail est réalisée en une matière présentant une dureté comprise entre 20 et 70 shore.

Selon une forme de réalisation de l'invention, l'appareil de massage selon l'invention comprend une seule tête de travail dont le centre est décalé par rapport au centre de la zone de travail.

Selon une variante de cette caractéristique, le centre de la tête de travail est décalé par rapport au centre de la zone de travail d'une distance supérieure ou égale à 1/6 de la plus petite dimension de la zone de travail et inférieure à la moitié de la plus petite dimension de la zone de travail.

Selon une autre forme de réalisation de l'invention, l'appareil de massage comprend au moins deux têtes de travail dont le centre est décalé par rapport au centre de la zone de travail.

Selon une variante de cette forme de réalisation, les moyens de manoeuvre sont adaptés pour déplacer chaque tête de travail en rotation autour d'un axe de rotation décalé par rapport au centre de la surface de travail correspondante et distinct de l'axe de rotation de la ou des autres têtes de travail. Selon cette variante, chaque tête de travail aura donc un mouvement propre en rotation.

Selon une caractéristique de l'invention, l'élément d'appui est creux et rigide. Le caractère rigide de l'élément d'appui doit être entendu comme le fait que l'élément d'appui ne se déforme pas sous un effort manuel d'application sur la peau. Cette caractéristique permet de contrôler la hauteur de saillie de chaque surface de travail par rapport au plan d'appui défini par la surface d'appui portée par l'élément d'appui. Dans le cas où la surface d'appui est formée par une matière souple cette dernière sera de préférence choisie pour que sa déformation induise une variation de la position du plan d'appui inférieure à quelques millimètres et de préférence inférieure à 2 mm.

Selon une caractéristique de l'invention, l'appareil de massage comprend des moyens d'application d'un courant électrique comprenant au moins une électrode qui est destinée à être en contact avec la peau et qui est raccordée à une unité de génération d'un courant et/ou d'une tension électriques. La mise en oeuvre d'une telle électrode permet d'induire par exemple des micro courants dans la peau, ou une électrostimulation des muscles, ou encore des phénomènes d'électrophorèse qui favorisent la pénétration de principes actifs appliqués sur la peau préalablement et/ou pendant le massage.

Selon une variante de cette caractéristique, au moins l'élément d'appui ou la tête de travail porte au moins une électrode.

Selon une forme de réalisation de l'invention, l'appareil de massage comprend des moyens de diffusion de lumière, en direction du visage. La mise en oeuvre de tels moyens de diffusion de lumière permet d'effectuer un traitement de photothérapie et/ou d'activer des principes actifs appliqués sur la peau préalablement et/ou pendant le massage.

Selon une caractéristique de cette forme de réalisation, les moyens de diffusion de lumière comprennent au moins une source de lumière et au moins un système optique de diffusion comprenant une face de sortie destinée à être orientée vers le visage.

Selon une variante de cette caractéristique, au moins l'élément d'appui ou la tête de travail comprend une face de sortie de la lumière.

Selon une variante, au moins l'élément d'appui ou la zone de travail est en partie au moins transparent.

Selon une autre forme de réalisation de l'invention, l'appareil de massage comprend des moyens d'application de produit cométique. La mise en oeuvre de tels moyens d'application permet de déposer sur la peau un produit cosmétique préalablement et/ou pendant le massage.

Selon une caractéristique de cette forme de réalisation, les moyens d'application de produit cosmétique comprennent au moins un capuchon qui comprend un tampon imbibé de produit cosmétique et qui est adapté de manière amovible sur l'élément d'appui et/ou la tête de travail.

Selon une autre caractéristique de cette forme de réalisation, les moyens d'application de produit cosmétique comprennent un réservoir de produit cosmétique et au moins une buse de distribution raccordée à un système de prélèvement de produit dans le réservoir, par exemple une pompe.

Selon encore une autre caractéristique de cette forme de réalisation, les moyens d'application de produit cosmétique comprennent au moins une buse de distribution située dans la tête de travail ou dans l'élément d'appui.

Selon une caractéristique de l'invention, la tête de massage est adaptée de manière amovible sur le boitier d'entrainement. Le caractère amovible de la tête de massage permet d'utiliser plusieurs têtes de massage interchangeables avec un même boitier.

Selon une variante de cette caractéristique, la tête de massage comprend des moyens d'identification et le boiter d'entrainement comprend des moyens de reconnaissance des moyens d'identification raccordés à une unité de commande adaptée pour contrôler le fonctionnement de l'appareil de massage en fonction de la tête de massage reconnue. La mise en oeuvre d'un tel système d'identification permet d'automatiser le réglage du fonctionnement de l'appareil de massage de sorte que l'utilisateur n'a pas à s'en préoccuper.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

Par ailleurs, diverses autres caractéristiques de l'invention ressortent de la description annexée effectuée en référence aux dessins qui illustrent des formes non limitatives de réalisation d'un appareil de massage selon l'invention.
- La figure 1 est une coupe schématique d'un appareil de massage selon l'invention.
- La figure 2 est une vue en perspective d'une première tête de massage,
- La figure 3 est une coupe de la tête de massage de la figure 2,
- La figure 4 est une coupe d'une deuxième tête de massage,
- La figure 5 est une coupe d'une troisième tête de massage.

Il est à noter que sur ces figures les éléments structurels et/ou fonctionnels communs aux différentes variantes peuvent présenter les mêmes références.

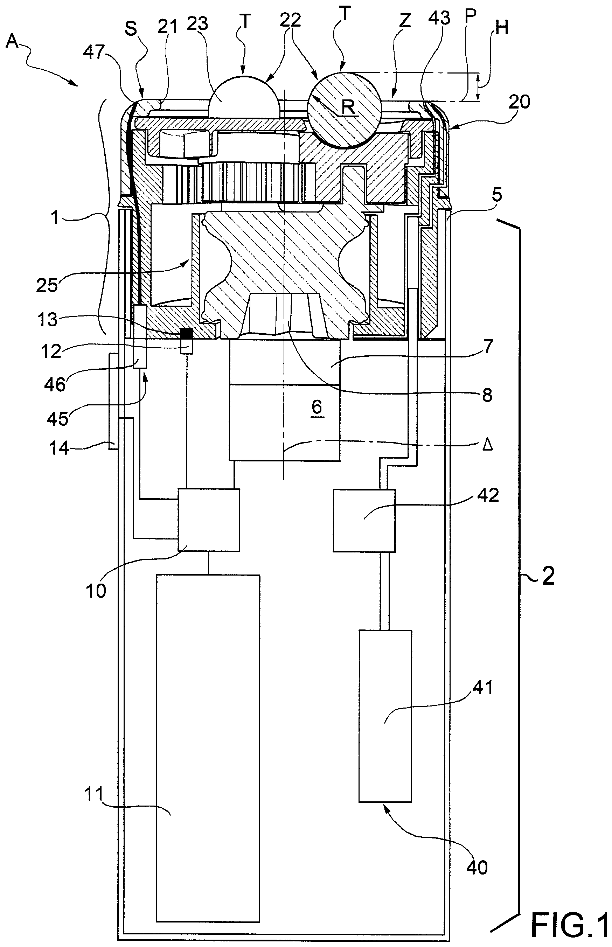

Un appareil de massage selon l'invention, tel qu'illustré à la figure 1 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage entraînés par un moteur électrique.

À cet effet, le boîtier d'entraînement 2 comprend un corps allongé de forme générale cylindrique qui comprend au niveau d'une de ses extrémités des moyens d'adaptation 5 de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie 11 disposé à l'intérieur du corps. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 14 accessible depuis l'extérieur du corps. L'interface de commande manuelle 14 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique, ou encore sous la forme d'un système d'identification magnétique mettant en oeuvre des aimants permanents et des interrupteurs à lame souple (ILS).

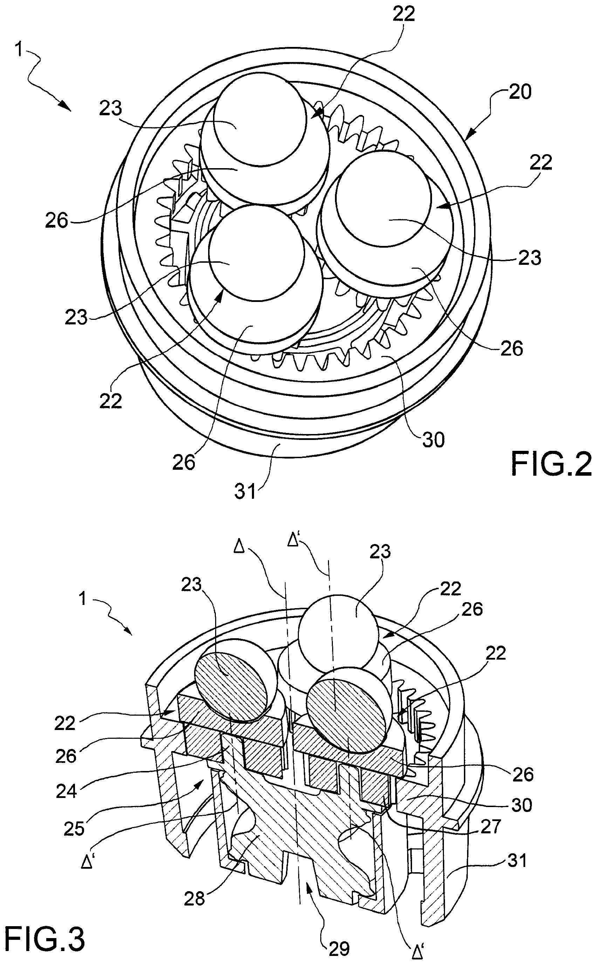

Selon l'invention, la tête de massage 1 est conçue pour réaliser un massage par déplacement d'une tête de travail contre la peau, la tête restant en contact permanent avec la peau. À cet effet, la tête de massage 1 comprend, comme le montre la figure 2 un élément d'appui 20 qui est destiné à venir contre le visage. L'élément d'appui 20 présente une forme générale annulaire et forme une couronne d'appui 21 qui définit une surface d'appui S s'inscrivant dans un plan d'appui P, ladite surface d'appui S étant conçue pour pouvoir épouser entièrement la peau si l'utilisateur le souhaite. Selon l'exemple illustré, l'élément d'appui 20 est creux et rigide tandis que la surface d'appui S est formée par une matière élastiquement déformable telle qu'un élastomère (par exemple en silicone ou en EPDM). Ainsi, la surface d'appui S peut se déformer de quelques millimètres, de préférence moins de deux, afin d'épouser la forme de la région du visage contre laquelle elle est appliquée.

La couronne d'appui 21 délimite une zone de travail Z à l'intérieur de laquelle se trouvent au moins, selon l'exemple illustré, trois têtes de travail 22 qui possèdent chacune une surface de travail T s'étendant en saillie du plan d'appui P. Selon l'exemple illustré, chaque tête de travail 22 comprend une bille 23 de sorte de la surface de travail T correspondante possède une forme convexe et plus particulièrement sphérique. Chaque surface de travail T s'étend alors, de manière permanente, en saillie du plan d'appui P d'une hauteur H, mesurée entre le sommet de la surface de travail T et le plan d'appui P, de préférence égale ou supérieure au rayon de courbure R de ladite bille. Selon une variante, La hauteur H de saillie de chaque surface de travail T est supérieure à la moitié du rayon de courbure R mais inférieur au rayon de courbure R.

Selon l'exemple illustré, chaque bille 23 appartenant à une tête de travail 22 est réalisée en une matière rigide telle que du métal. Ainsi, chaque surface de travail T est rigide par opposition au caractère souple ou élastiquement déformable de la surface d'appui S. De plus, selon cet exemple la surface de chaque bille 23 est lisse.

Selon l'invention, la tête de massage 1 comprend également des moyens de manoeuvre 25 adaptés pour déplacer chaque tête de travail 22 en rotation selon un axe Δ décalé par rapport au centre de chaque surface de travail T correspondante. Les moyens de manoeuvre 25 sont alors adaptés pour coopérer avec les moyens d'entraînement 7 et plus particulièrement avec l'arbre de sortie 8 de manière à transmettre et transformer le mouvement de rotation du moteur électrique 6 en un mouvement de rotation des têtes de travail 22.

Selon l'exemple illustré et comme cela ressort des figures 2 et 3, les moyens de manoeuvre 25 sont adaptés pour assurer un mouvement planétaire aux trois billes 23, c'est-à-dire un mouvement de rotation principal des trois billes 23 autour de l'axe de rotation Δ associé à un mouvement de rotation secondaire de chacune des billes autour d'un axe de rotation secondaire Δ' qui se déplace en rotation autour de l'axe de rotation Δ lors du fonctionnement de l'appareil de massage selon l'invention.

À cet effet, chaque tête de travail 22 comprend un disque 26 qui est centré par rapport à l'axe de rotation secondaire Δ' et qui porte sur sa face supérieure la bille 23 correspondante liée de manière rigide audit disque 26. Chaque tête de travail comprend en outre un pignon planétaire 27 qui est lié de manière rigide à la face inférieure du disque 26 correspondant et qui est coaxial à l'axe de rotation secondaire Δ'.

Les moyens de manoeuvre 25 comprennent alors un arbre de manoeuvre 28 d'axe Δ qui comprend sur une face intérieure un logement 29 de réception de l'arbre de sortie 8 et qui porte sur une face supérieure trois pions 24 engagés chacun dans un alésage axial d'un pignon planétaire 27. Les moyens de manoeuvre comprennent en outre une couronne périphérique fixe 30 liée de manière rigide à un carter 31 constitutif de la tête de massage 1. Chacun des pignons planétaires 27 engrène la couronne périphérique fixe 30 de sorte que la rotation de l'arbre de manoeuvre 28 entraîne le mouvement planétaire des billes 23 décrit précédemment.

Dans cette variante illustrée, on peut inverser les matières utilisées pour les billes et pour la couronne d'appui, c'est-à-dire, billes en matière souple et la couronne en matière rigide.

En fonction du besoin et dans le but d'atténuer le pétrissage, on peut aussi construire la tête de massage 1 de façon à ce que chaque tête de travail 22 soit montée sur l'axe Δ' et à ce qu'elle exerce une rotation régulière sur elle-même.

L'appareil de massage ainsi constitué est mis en oeuvre de la manière suivante. La surface d'appui S est placée contre le visage puis l'utilisateur met en marche l'appareil de massage A au moyen de l'interface de commande manuel 14, les têtes de travail 22 s'animent alors d'un mouvement de rotation planétaire alors que la peau autour de la zone de travail Z est maintenue par la surface d'appui S de forme annulaire.

Le massage réalisé au moyen de l'appareil selon l'invention permet notamment de pétrir la peau du visage en profondeur et de réduire ainsi les rides en stimulant la circulation sanguine et en relançant la production des éléments constitutifs de la peau.

Afin d'optimiser ce traitement, l'appareil de massage A tel qu'illustré à la figure 1 comprend des moyens 40 d'application de produit cosmétique. Selon l'exemple illustré, les moyens d'application de produit cosmétique 40 comprennent un réservoir 41 situé dans le boitier d'entraînement 2 et raccordé, via un système de prélèvement 42, par exemple une pompe, à une buse de distribution 43 située dans l'élément d'appui 20. La pompe de prélèvement 42 est pilotée par l'unité de commande 10 de manière à assurer la distribution de produit cosmétique lors du fonctionnement de l'appareil de massage A. Bien entendu, l'élément d'appui 20 pourrait comprendre plus d'une buse de distribution. De plus, chaque tête de travail pourrait également comprendre une buse de distribution de produit cosmétique qui serait alimentée à partir d'un réservoir souple aménagé dans la tête de travail correspondante et sollicité par un système de came lors de la rotation de ladite tête de travail.

Par ailleurs, toujours selon l'exemple illustré aux figures 1 à 3, l'appareil de massage A comprend aussi des moyens d'application d'un courant électrique 45 qui comprennent une unité 46 de génération d'un courant et/ou d'une tension électriques. L'unité de génération 46 est pilotée par l'unité de commande 10. L'unité de génération 46 est raccordée à une électrode 47 portée par l'élément d'appui 20.

Lors de l'utilisation de l'appareil de massage A, l'unité de commande 10 pilote le fonctionnement de l'unité de génération 46 de sorte que lorsque l'électrode 47 est en contact avec la peau un phénomène d'électrophorèse est induit qui favorise l'assimilation des principes actifs du produit cosmétique.

Selon l'invention, les têtes de travail 22 ne sont pas nécessairement animées d'un mouvement de type planétaire tel que décrit précédemment.

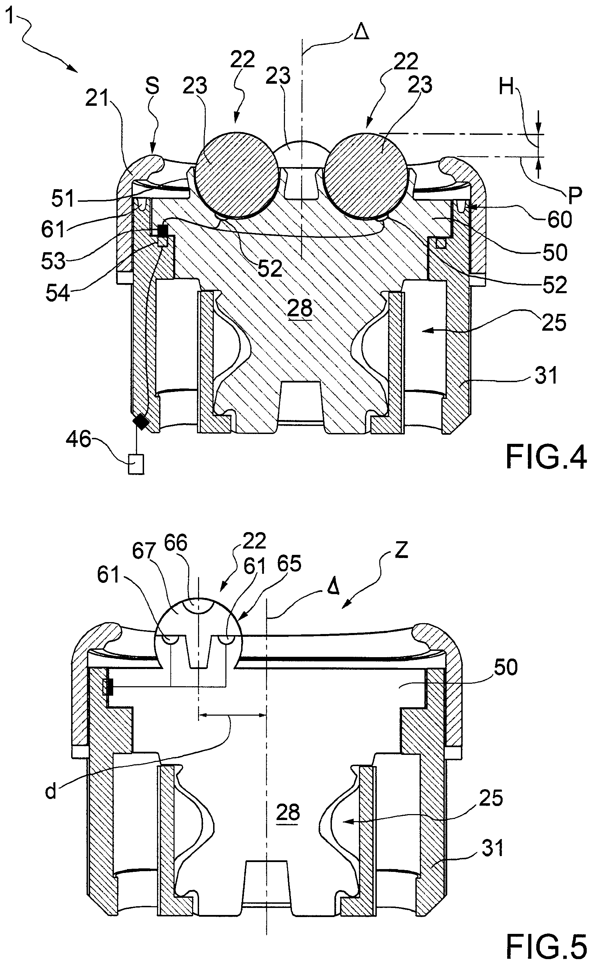

Ainsi, la figure 4 illustre une variante de réalisation d'une tête de massage pour un appareil de massage selon l'invention qui diffère de celle décrite en relation avec les figures 1 à 3, en ce que les trois têtes de travail 22 sont portées par un plateau circulaire 50 lié en rotation à l'arbre de manoeuvre 28. Le plateau 50 porte les trois têtes de travail 22 formées par les billes de métal 23 dont les centres sont décalés par rapport à l'axe de rotation Δ. Par ailleurs, les billes 23 sont chacune enfermée dans une coupelle hémisphérique 51 liée rigidement au plateau 50. Ainsi, chaque bille 23 est mobile en rotation sur elle-même de manière qu'elle peut rouler sur la peau lors de l'utilisation de l'appareil de massage selon cette variante de réalisation.

De plus, selon cet exemple de réalisation, chaque tête de travail comprend une électrode d'électrostimulation formée par la bille 23 correspondante qui est raccordée électriquement à l'unité de génération d'un courant 46 via un système de balais 52, 53 et d'une piste annulaire 54.

Toujours selon cet exemple de réalisation, la tête de massage comprend des moyens 60 de diffusion de lumière en direction du visage. Dans le cas présent les moyens de diffusion 60 sont aménagés dans la tête de massage et comprennent en tant que source de lumière, des diodes électroluminescentes 61 pilotées par l'unité de commande 10. Les sources de lumière 61 sont alors associées à un système optique formé par l'élément d'appui qui est transparent et qui comprend une face de sortie de la lumière située au niveau de la surface d'appui S et donc destinée à être orientée vers le visage de l'utilisateur de l'appareil de massage A selon l'invention.

Pour ce faire, toute la zone de travail (Z) peut être constituée de pièces transparentes afin de pouvoir faire passer la lumière.

La figure 4 illustre encore une autre variante de réalisation d'une tête de massage 1 d'un appareil de massage A conforme à l'invention. Selon cette variante, la tête de massage 1 ne comprend qu'une seule tête de travail 22 de forme sphérique qui est liée de manière rigide au plateau 50 en étant décalée par rapport à son centre. Selon cet exemple de réalisation, le centre de la tête de travail 22 est décalé par rapport au centre de la zone de travail Z par lequel passe l'axe Δ d'une distance d supérieure ou égale à 1/6 de la plus petite dimension de la zone de travail Z, ici le diamètre de la zone de travail Z, et inférieure à la moitié de ladite plus petite dimension de la zone de travail Z.

Selon cet exemple de réalisation, la tête de travail 22 porte un capuchon amovible 65 qui forme une surface de travail correspondante et comprend au niveau de son sommet un tampon 66 imbibé de produit cosmétique. Le capuchon amovible 65 forme ainsi un moyen d'application de produit cosmétique.

Selon cet exemple de réalisation, le capuchon 65 comprend en outre un système optique 67 de diffusion de la lumière produit par les sources de lumière 61 situées dans la tête de travail 22. Le système optique 67 est ici formé par une calotte sphérique en matériau transparent qui assure une fonction de guide de lumière.

Bien entendu, diverses autres modifications ou variantes de l'appareil et de la tête de massage selon l'invention, peuvent être envisagées dans le cadre des revendications annexées.

## Revendications

1. Appareil de massage pour le visage comprenant :
- une tête de massage (1) qui comprend :
- un élément d'appui (20) présentant une forme générale annulaire qui est destiné à venir contre le visage et qui forme une couronne d'appui (21) définissant, d'une part, une surface d'appui (S) s'inscrivant dans un plan d'appui (P) et, d'autre part, une zone de travail (Z) située à l'intérieur de la couronne d'appui,
- à l'intérieur de la couronne d'appui et dans la zone de travail (Z), au moins une tête de travail (22) qui possède une surface de travail s'étendant en saillie du plan d'appui (P),
- des moyens de manoeuvre (25) adaptés pour déplacer en rotation chaque tête de travail (22) selon au moins un axe de rotation (Δ, Δ') décalé par rapport au centre de la surface de travail (T) correspondante,
- et un boitier d'entraînement (2) qui porte la tête de massage (1) et qui comprend un moteur électrique (6) actionnant des moyens d'entrainement (7) adaptés pour transmettre le mouvement du moteur électrique aux moyens de manoeuvre (25), appareil pour lequel en outre chaque surface de travail s'étend, de manière permanent en saillie du plan d'appui (P) d'une hauteur H, mesurée entre le sommet de la surface de travail et le plan d'appui (P).

2. Appareil de massage selon la revendication précédente, **caractérisé en ce que** la surface d'appui (S) est constituée d'une première matière et chaque surface de travail (T) est constituée d'une seconde matière de rigidité différente de celle de la première matière, la première matière et la seconde matière étant choisies parmi :
- une matière rigide, non déformable sous un effort manuel,
- une matière élastiquement déformable sous un effort manuel.

3. Appareil de massage selon la revendication précédente, **caractérisé en ce que** la surface de travail (T) est constituée d'une matière élastiquement déformable.

4. Appareil de massage selon l'une des revendications précédentes, **caractérisé en ce que** chaque surface de travail (T) possède une forme convexe

5. Appareil de massage selon la revendication précédente, **caractérisé en ce que** chaque surface de travail (T) possède une forme substantiellement sphérique et s'étend, de manière permanente, en saillie du plan d'appui (P) d'une hauteur (H), mesurée entre le sommet de la surface de travail (T) et le plan d'appui (P), égale ou supérieure au rayon de courbure (R) de la surface de travail (T).

6. Appareil de massage selon l'une des revendications précédentes, **caractérisé en ce que** chaque tête de travail (22) comprend une bille (23) qui forme la surface de travail (T) correspondante.

7. Appareil de massage selon la revendication 6, **caractérisé en ce que** chaque bille (23) est mobile en rotation sur elle-même.

8. Appareil de massage selon l'une des revendications précédentes, **caractérisé en ce qu'**il comprend une seule tête de travail (22) dont le centre est décalé par rapport au centre de la zone de travail (Z).

9. Appareil de massage selon la revendication précédente, **caractérisé en ce que** le centre de la tête de travail (22) est décalé par rapport au centre de la zone de travail (Z) d'une distance (d) supérieure ou égale à 1/6 de la plus petite dimension de la zone de travail (Z) et inférieure à la moitié de la plus petite dimension de la zone de travail (Z).

10. Appareil de massage selon l'une des revendications 1 à 7, **caractérisé en ce qu'**il comprend au moins deux têtes de travail (22) dont le centre est décalé par rapport au centre de la zone de travail (Z).

11. Appareil de massage selon la revendication précédente, **caractérisé en ce que** les moyens de manoeuvre (25) sont adaptés pour déplacer chaque tête de travail (22) en rotation autour d'un axe de rotation (Δ,Δ') décalé par rapport au centre de la surface de travail (T) correspondante et distinct de l'axe de rotation (Δ,Δ') de la ou des autres têtes de travail.

12. Appareil de massage selon l'une des revendications précédentes, **caractérisé en ce que** l'élément d'appui (20) est creux et rigide.

13. Appareil de massage selon l'une des revendications précédentes, **caractérisé en ce qu'**il comprend des moyens (60) de diffusion de lumière.

14. Appareil de massage selon la revendication précédente, **caractérisé en ce que** les moyens de diffusion de lumière (60) comprennent au moins une source de lumière (61) et au moins un système optique (67) de diffusion comprenant une face de sortie destinée à être orientée vers le visage.

15. Appareil de massage selon la revendication précédente, **caractérisé en ce qu'**au moins l'élément d'appui (20) ou la tête de travail comprend une face de sortie de la lumière.

16. Appareil de massage selon l'une des deux revendications précédentes, **caractérisé en ce qu'**au moins l'élément d'appui (20) ou la zone de travail est en partie au moins transparent.

17. Appareil de massage selon l'une des revendications précédentes, **caractérisé en ce que** la tête de massage (1) est adaptée de manière amovible sur le boitier d'entrainement (2).

18. Appareil de massage selon la revendication précédente, **caractérisé en ce que** la tête de massage (1) comprend des moyens d'identification (13) et **en ce que** le boitier d'entrainement (2) comprend des moyens de reconnaissance (12) des moyens d'identification (13) raccordés à une unité de commande (10) adaptée pour contrôler le fonctionnement de l'appareil de massage en fonction de la tête de massage (1) reconnue.

## Patentansprüche

1. Gerät zur Massage für das Gesicht, das umfasst:
- einen Massagekopf (1), der umfasst:
- ein Auflageelement (20), das im Wesentlichen eine Ringform aufweist, dazu bestimmt ist, an dem Gesicht anzuliegen, und einen Auflagering (21) bildet, der einerseits eine Auflagefläche (S), die sich in einer Auflageebene (P) befindet, und andererseits einen Arbeitsbereich (Z) definiert, der sich im Innern des Auflagerings befindet,
- innerhalb des Auflagerings und in dem Arbeitsbereich (Z) mindestens einen Arbeitskopf (22), der eine Arbeitsfläche besitzt, die von der Auflageebene (P) hervorsteht,
- Betätigungsmittel (25), die eingerichtet sind, jeden Arbeitskopf (22) um mindestens eine Drehachse (Δ, Δ'), die mit Bezug auf das Zentrum der entsprechenden Arbeitsfläche (T) versetzt ist, rotatorisch zu bewegen,
- und ein Antriebsgehäuse (2), das den Massagekopf (1) trägt und das einen elektrischen Motor (6) umfasst, der von den Antriebsmitteln (7) betätigt wird, die eingerichtet sind, die Bewegung des Elektromotors auf die Betätigungsmittel (25) zu übertragen, wobei für das Gerät ferner jede Arbeitsfläche permanent über die Auflageebene (P) um eine Höhe H hervorsteht, die zwischen dem Höhepunkt der Arbeitsfläche und der Auflageebene (P) gemessen wird.

2. Gerät zur Massage nach dem vorhergehenden Anspruch, **dadurch gekennzeichnet, dass** die Auflagefläche (S) aus einem ersten Material besteht, und dass jede Arbeitsfläche (T) aus einem zweiten Material mit anderer Steifigkeit als das erste Material besteht, wobei das erste Material und das zweite Material ausgewählt sind aus:
- einem starren Material, das nicht unter einer manuellen Kraft verformbar ist,
- einem Material, das unter einer manuellen Kraft elastisch verformbar ist.

3. Gerät zur Massage nach dem vorhergehenden Anspruch, **dadurch gekennzeichnet, dass** die Arbeitsfläche (T) aus einem elastisch verformbaren Material besteht.

4. Gerät zur Massage nach einem der vorhergehenden Ansprüche, **dadurch gekennzeichnet, dass** jede Arbeitsfläche (T) eine konvexe Form besitzt.

5. Gerät zur Massage nach dem vorhergehenden Anspruch, **dadurch gekennzeichnet, dass** jede Arbeitsfläche (T) eine im wesentlichen kugelförmige Form besitzt und permanent über die Auflageebene (P) um eine Höhe (H), die zwischen dem Höhepunkt der Arbeitsfläche (T) und der Auflagefläche (P) gemessen wird, mit gleichem oder größeren Krümmungsradius (R) als die Arbeitsfläche (T) hervorsteht.

6. Gerät zur Massage nach einem der vorhergehenden Ansprüche, **dadurch gekennzeichnet, dass** jeder Arbeitskopf (22) eine Kugel (23) umfasst, die die entsprechende Arbeitsfläche (T) bildet.

7. Gerät zur Massage nach Anspruch 6, **dadurch gekennzeichnet, dass** jede Kugel (23) um sich selbst rotatorisch bewegbar ist.

8. Gerät zur Massage nach einem der vorhergehenden Ansprüche, **dadurch gekennzeichnet, dass** es einen Arbeitskopf (22) aufweist, dessen Zentrum von dem Zentrum des Arbeitsbereichs (Z) versetzt ist.

9. Gerät zur Massage nach dem vorhergehenden Anspruch, **dadurch gekennzeichnet, dass** das Zentrum des Arbeitskopfes (22) mit Bezug auf das Zentrum des Arbeitsbereichs (Z) um einen Abstand (d) versetzt ist, der größer oder gleich 1/6 der kleinsten Abmessung des Arbeitsbereichs (Z) und kleiner als die Hälfte der kleinsten Abmessung des Arbeitsbereichs (Z) ist.

10. Gerät zur Massage nach einem der Ansprüche 1 bis 7, **dadurch gekennzeichnet, dass** es mindestens zwei Arbeitsköpfe (22) umfasst, deren Zentrum von dem Zentrum des Arbeitsbereichs (Z) versetzt ist.

11. Gerät zur Massage nach dem vorhergehenden Anspruch, **dadurch gekennzeichnet, dass** die Betätigungsmittel (25) eingerichtet sind, jeden Arbeitskopf (22) rotatorisch um eine Drehachse (Δ, Δ') zu bewegen, die mit Bezug auf das Zentrum der entsprechenden Arbeitsfläche (T) versetzt ist und sich von der Drehachse (Δ, Δ') des oder der anderen Arbeitsköpfe unterscheidet.

12. Gerät zur Massage nach einem der vorhergehenden Ansprüche, **dadurch gekennzeichnet, dass** das Auflageelement (20) hohl und starr ist.

13. Gerät zur Massage nach einem der vorhergehenden Ansprüche, **dadurch gekennzeichnet, dass** es Mittel (60) zur Streuung von Licht umfasst.

14. Gerät zur Massage nach dem vorhergehenden Anspruch, **dadurch gekennzeichnet, dass** die Mittel zur Streuung von Licht (60) mindestens eine Lichtquelle (61) und mindestens ein optisches System (67) zur Streuung umfassen, das eine Austrittsfläche umfasst, die dazu bestimmt ist, auf das Gesicht ausgerichtet zu werden.

15. Gerät zur Massage nach dem vorhergehenden Anspruch, **dadurch gekennzeichnet, dass** mindestens das Auflageelement (20) oder der Arbeitskopf eine Austrittsfläche für das Licht aufweist.

16. Gerät zur Massage nach einem der beiden vorhergehenden Ansprüche, **dadurch gekennzeichnet, dass** mindestens das Auflageelement (20) oder der Arbeitsbereich mindestens teilweise transparent ist.

17. Gerät zur Massage nach einem der vorhergehenden Ansprüche, **dadurch gekennzeichnet, dass** der Massagekopf (1) abnehmbar an dem Antriebsgehäuse (2) angebracht ist.

18. Gerät zur Massage nach dem vorhergehenden Anspruch, **dadurch gekennzeichnet, dass** der Massagekopf (1) Mittel zur Identifikation (13) umfasst, und dass das Antriebsgehäuse (2) Mittel zur Erkennung (12) der Mittel zur Identifikation (13) umfasst, die mit einer Steuereinheit (10) verbunden sind, die eingerichtet ist, den Betrieb des Geräts zur Massage als Funktion des erkannten Massagekopfes (1) zu steuern.

## Claims

1. Facial massage device comprising:
- a massage head (1) that comprises:
- a bearing element (20) having an overall ring shape that is intended to be placed against the face and that forms a bearing ring (21) defining a bearing surface (S) lying within a bearing plane (P) on the one hand, and a working area (Z) located within the bearing ring on the other hand,
- within the bearing ring and inside the working area (Z), at least one working head (22) that has a working surface that extends, protruding, from the bearing plane (P),
- manoeuvring means (25) designed to move each working head (22) in rotation about at least one axis of rotation (Δ, Δ') offset relative to the centre of the corresponding working surface (T),
- and a drive housing (2) that carries the massage head (1) and that comprises an electric motor (6) that actuates drive means (7) designed to transmit the motion from the electric motor to the manoeuvring means (25),
device wherein furthermore, each working surface extends permanently protruding from the bearing plane (P) by a height H, measured between the top of the working surface and the bearing plane (P).

2. Massage device according to the previous claim, **characterised in that** the bearing surface (S) is composed of a first material and that each working surface (T) is composed of a second material having a rigidity that is different from that of the first material, the first material and the second material being chosen from among:
- a rigid material that is non-deformable under a manual force,
- a material that is elastically deformable under a manual force.

3. Massage device according to the previous claim, **characterised in that** the working surface (T) is composed of an elastically deformable material.

4. Massage device according to one of the previous claims, **characterised in that** each working surface (T) has a convex shape.

5. Massage device according to the previous claim, **characterised in that** each working surface (T) has a substantially spherical shape and extends, permanently protruding from the bearing plane (P) by a height (H) measured between the top of the working surface (T) and the bearing plane (P) that is greater than or equal to the curvature radius (R) of the working surface (T).

6. Massage device according to one of the previous claims, **characterised in that** each working head (22) comprises a ball (23) that forms the corresponding working surface (T).

7. Massage device according to claim 6, **characterised in that** each ball (23) is capable of moving in rotation about itself.

8. Massage device according to one of the previous claims, **characterised in that** it comprises a single working head (22), the centre of which is offset relative to the centre of the working area (Z).

9. Massage device according to the previous claim, **characterised in that** a centre of the working head (22) is offset relative to the centre of the working area (Z) by a distance (d) greater than or equal to ¹/₆ of the smallest dimension of the working area (Z) and less than half of the smallest dimension of the working area (Z).

10. Massage device according to one of claims 1 to 7, **characterised in that** it comprises at least two working heads (22), the centre of which is offset relative to the centre of the working area (Z).

11. Massage device according to the previous claim, **characterised in that** the manoeuvring means (25) are designed to move each working head (22) in rotation about an axis of rotation (Δ, Δ') offset relative to the centre of the corresponding working surface (T) and separate from the axis of rotation (Δ, Δ') of the one or more other working heads.

12. Massage device according to one of the previous claims, **characterised in that** the bearing element (20) is hollow and rigid.

13. Massage device according to one of the previous claims, **characterised in that** it further comprises light diffusion means (60).

14. Massage device according to the previous claim, **characterised in that** the light diffusion means (60) comprise at least one light source (61) and at least one optical diffusion system (67) comprising an outlet side intended to be oriented towards the face.

15. Massage device according to the previous claim, **characterised in that** at least the bearing element (20) or the working head comprises a light outlet side.

16. Massage device according to one of the two previous claims, **characterised in that** at least the bearing element (20) or the working area is at least partially transparent.

17. Massage device according to one of the previous claims, **characterised in that** the massage head (1) is fitted in a removable manner on the drive housing (2).

18. Massage device according to the previous claim, **characterised in that** the massage head (1) comprises identification means (13) and **in that** the drive housing (2) comprises recognition means (12) for recognising the identification means (13) that are connected to a control unit (10) designed for controlling the functioning of the massage device on the basis of the recognised massage head (1).
